# EUROPEAN PATENT APPLICATION

(11) **EP 1 576 878 A1**
(43) Date of publication of application: **21.09.2005**
(21) Application number: 05005850.2
(22) Date of filing: 17.03.2005
(51) Int. Cl.: A01K 67/027, C12N 15/11

(54) **Glyt1 transgenic mice**

(30) Priority: 19.03.2004 EP 04101156
(71) Applicant: F. HOFFMANN-LA ROCHE AG, 4070 Basel (CH)
(72) Inventor: Alberati-Giani, Daniela, 4800 Zofingen (CH); Pauly-Evers, Meike, 79111 Freiburg (DE)

(57) **Abstract**

The present invention provides genetic constructs and methods for producing transgenic non-human animals comprising within their genome transgenic DNA encoding GLYT1. These transgenic animals could be further used to generate transgenic animals which produce more active GLYT1. Moreover, said transgenic animals producing more GLYT1 protein, as well as the methods of producing them are also provided. The invention also relates to the use of these animals as a model for analyzing the effects of depressing synaptic NMDA receptor function and studying the ability of compounds to reduce symptoms of psychotic behavior.

## Description

Glycine is the major inhibitory neurotransmitter in the spinal cord and brainstem and is also a co-agonist at the NMDA receptor. The extracellular concentration of glycine is regulated by at least two Na⁺/Cl⁻-dependent glycine transporters (GLYT1 and GLYT2) which play an important role in the termination of post-synaptic glycinergic actions and maintenance of low extracellular glycine concentration by re-uptake of glycine into presynaptic nerve terminals and surrounding fine glial processes. GLYT2 is expressed at high levels in the rodent spinal cord, brainstem and cerebellum where its expression correlates very well with the presence of strychnine-sensitive glycine receptors (Zafra, F., et al., J Neurosci, 1995. 15(5 Pt 2): p. 3952-69; Luque, J.M., N. Nelson, and J.G. Richards, Neuroscience, 1995. 64(2): p. 525-35 and Jursky, F. and N. Nelson, J Neurochem, 1995. 64(3): p. 1026-33]. Immunohistochemical analysis suggests a predominantly pre-synaptic localization in presumptive glycinergic synapses (Zafra, F., et al., J Neurosci, 1995. 15(5 Pt 2): p. 3952-69; Spike, R.C., et al., Neuroscience, 1997. 77(2): p. 543-51), strongly suggesting a role in the termination of glycinergic inhibitory synaptic transmission. Human GLYT2 has been cloned and appears to exhibit a similar expression pattern (Morrow, J.A., et al., FEBS Lett, 1998. 439(3): p. 334-40). GLYT2 has some degree of heterogeneity. Indeed two GLYT2 isoforms (2a and 2b) have been identified in rodent brains.

GLYT1 can be distinguished pharmacologically from GLYT2 by its sensitivity to be blockaded by sarcosine and N-methylated derivative of glycine (Liu, Q.R., et al., J Biol Chem, 1993. 268(30): p. 22802-8, Kim, K.M., et al., Mol Pharmacol, 1994. 45(4): p. 608-17). The human *glyt1* gene has been cloned and encodes four isoforms GLYT1a, 1b, 1c and 1d (Kim, K.M., et al., Mol Pharmacol, 1994. 45(4): p. 608-17) whereas only two rat isoforms, GLYT1a and 1b, have been identified (Guastella, J., et al., Proc Natl Acad Sci USA, 1992. 89(15): p. 7189-93; Smith, K.E., et al., Neuron, 1992. 8(5): p. 927-35; Borowsky, B., E. Mezey, and B.J. Hoffman, Neuron, 1993. 10(5): p. 851-63). GLYT1 appears to be expressed in both glia and neurons in the rat CNS (Zafra, F., et al., J Neurosci, 1995. 15(5 Pt 2): p. 3952-69; Smith, K.E., et al., Neuron, 1992. 8(5): p. 927-35; Borowsky, B., E. Mezey, and B.J. Hoffman, Neuron, 1993. 10(5): p. 851-63; Zafra, F., et al., Eur J Neurosci, 1995. 7(6): p. 1342-52), with GLYT1a apparently expressed in the gray matter as well as in some peripheral tissues whilst GLYT1b is expressed only in the white matter of the CNS (Borowsky, B., E. Mezey, and B.J. Hoffman, Neuron, 1993. 10(5): p. 851-63). In humans, a probe common to all GLYT1 isoforms revealed expression in several peripheral tissues, most notably the kidney, whereas GLYT1c seems to be brain specific (Kim, K.M., et al., Mol Pharmacol, 1994. 45(4): p. 608-17). The GLYT1 isoforms differ only in their amino termini and 5' non-coding regions (Kim, K.M., et al., Mol Pharmacol, 1994. 45(4): p. 608-17; Borowsky, B., E. Mezey, and B.J. Hoffman, Neuron, 1993. 10(5): p. 851-63). GLYT1a and GLYT1b originate from transcription directed from alternate promoters whereas human GLYT1c is a splice variant of the GLYT1b transcript (Kim, K.M., et al., Mol Pharmacol, 1994. 45(4): p. 608-17; Adams, R.H., et al., J Neurosci, 1995. 15(3 Pt 2): p. 2524-32; Borowsky, B. and B.J. Hoffman, J Biol Chem, 1998. 273(44): p. 29077-85). The peripheral expression of GLYT1 and the differential CNS expression patterns of the isoforms are somewhat controversial with major discrepancies evident between the published studies. GLYT1 is expressed together with GLYT2 in the spinal cord, brainstem and diencephalon. Interestingly GLYT1 is expressed in forebrain areas such as the cortex, hippocampus and olfactory bulb where no functional inhibitory glycinergic neurons have been found (Zafra, F., et al., J Neurosci, 1995. 15(5 Pt 2): p. 3952-69; Guastella, J., et al., Proc Natl Acad Sci U S A, 1992. 89(15): p. 7189-93; Smith, K.E., et al., Neuron, 1992. 8(5): p. 927-35; Borowsky, B., E. Mezey, and B.J. Hoffman, Neuron, 1993 Eur J Neurosci, 1995. 7(6): p. 1342-52) thus, suggesting additional roles for GLYT1, which might include regulation of NMDA receptor-mediated neurotransmission (Smith, K.E., et al., Neuron, 1992. 8(5): p. 927-35).

Binding of both glutamate and glycine is necessary for NMDA receptor activation. Whilst glutamate is released in an activity-dependent manner from pre-synaptic terminals, glycine is apparently present at a more constant level, indicating a more modulatory function. Measurements of glycine concentration in the extracellular and cerebrospinal fluids suggest that it is present at low micromolar levels (Westergren, I. et al., J Neurochem, 1994. 62(1): p. 159-65). However glycine transporters might reduce the glycine concentration markedly in the local microenvironment of NMDA receptors. Indeed, expression of GLYT1b in *Xenopus* oocytes has been shown to reduce the glycine concentration at co-expressed NMDA receptors (Supplisson, S. and C. Bergman, J Neurosci, 1997. 17(12): p. 4580-90). Additionally, recent studies have suggested that glycine uptake mechanisms can regulate synaptic NMDA receptor activity (Berger, A.J., S. Dieudonne, and P. Ascher, J Neurophysiol, 1998. 80(6): p. 3336-40; Bergeron, R., et al., Proc Natl Acad Sci U S A, 1998. 95(26): p. 15730-4). NMDA receptor glycine affinity is influenced by the identity of the receptor NR2 subunit and in recombinant systems, receptors containing NR2A exhibit a markedly reduced affinity for glycine relative to those containing NR2B, C or D (Ikeda, K., et al., FEBS Lett, 1992. 313(1): p. 34-8; Kutsuwada, T., et al., Nature, 1992. 358(6381): p. 36-41; Priestley, T., et al., Mol Pharmacol, 1995. 48(5): p. 841-8). A population of NMDA receptors with a markedly lower affinity for glycine appears during maturation, paralleling the developmental increase in expression of NR2A (Kew, J.N., et al., J Neurosci, 1998. 18(6): p. 1935-43) suggesting the existence of a population of NMDA receptors not saturated by glycine under normal physiological conditions

Glutamate neurotransmission, in particular NMDA receptor activity, plays a critical role in synaptic plasticity, learning and memory, such as the NMDA receptors appears to serve as a graded switch for gating the threshold of synaptic plasticity and memory formation (Hebb, D., *The organization of behavior.* 1949, New York: Wiley; Bliss, T.V. and G.L. Collingridge, Nature, 1993. 361(6407): p. 31-9). Transgenic mice overexpressing the NMDA NR2B subunit exhibit enhanced synaptic plasticity and superior ability in learning and memory (Tang, Y.P., et al., Nature, 1999. 401(6748): p. 63-9).

NMDA receptor hypofunction has been implicated in the pathophysiology of schizophrenia (Olney, J.W. and N.B. Farber, Arch Gen Psychiatry, 1995. 52(12): p. 998-1007; Hirsch, S.R., et al., Pharmacol Biochem Behav, 1997. 56(4): p. 797-802). Non-competitive NMDA receptor antagonists such as PCP and ketamine can induce schizophrenia-like psychosis (Allen, R.M. and S.J. Young, Am J Psychiatry, 1978. 135(9): p. 1081-4; Javitt, D.C. and S.R. Zukin, Am J Psychiatry, 1991. 148(10): p. 1301-8; Krystal, J.H., et al., Arch Gen Psychiatry, 1994. 51(3): p. 199-214), which incorporates positive and negative symptoms as well as cognitive dysfunction, thus closely resembling schizophrenia in patients (Javitt, D.C., et al., Biol Psychiatry, 1999. 45(6): p. 668-79). Transgenic mice expressing reduced levels of the NMDAR1 subunit displays behavioral abnormalities similar to those observed in pharmacologically induced models of schizophrenia, supporting a model in which reduced NMDA receptor activity results in schizophrenia-like behavior (Mohn, A.R., et al., Cell, 1999. 98(4): p. 427-36). Furthermore mice lacking the NMDA receptor 2A subunit exhibit an increased spontaneous locomotor activity in a novel environments and an impairment of latent learning in a water-finding task besides deficit in hippocampal LTP and spatial learning (Miyamoto Y, Yamada K, Noda Y, Mori H, Mishina M and Nabeshima T, J. Neurosci. 2001 21(2): 750-757).

A mouse overproducing GLYT1 provides a valuable tool to assess the physiological function of GLYT1. These mice should exhibit decreased levels of glycine in the forebrain and they can be useful in addressing the question of whether active regulation of NMDA receptor glycine site occupancy is important for physiological NMDA receptor function.

The present invention provides genetic constructs and methods for producing transgenic non-human animals comprising within their genome transgenic DNA encoding GLYT1. These transgenic animals can be further used to generate transgenic animals which overexpress active GLYT1. Moreover, said transgenic animals overexpress GLYT1 protein, as well as the methods of producing them are also provided. The invention also relates to the use of these animals as a model for analyzing the effects of depressing synaptic NMDA receptor function and studying the ability of compounds to reduce symptoms of psychotic behavior.

The present invention therefore provides a genetic construct comprising a DNA sequence encoding GLYT1, operatively linked to a promoter. The sequence of *glyt1* gene may encode an isoform of GLYT1. Preferably, the sequence of *glyt1* gene encodes GLYT1b.

Preferably, the sequence of *glyt1* gene is a cDNA sequence. More preferably, the cDNA incorporates at least one intron sequence. Most preferably, the at least one intron sequence comprises a polyadenylation site.

The sequence of *glyt1* gene can be derived from any animal, preferably the sequence of *glyt1* derives from a mammal, more preferably the sequence of *glyt1* gene is a human sequence.

The promoter may be a neuronal promoter. In one embodiment the promoter is a tissue-specific promoter. A tissue-specific promoter may be any promoter, which controls and directs expression of a gene in a tissue-specific manner, e.g., in brain tissue, in muscle tissue, in liver tissue, in kidney tissue, etc. Preferably, the promoter provides specific expression in the forebrain. Most preferably, the promoter is the mouse CamKαII-promoter.

The promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su IJ, Chang TY; Lac/Tet dual-inducible system functions in mammalian cell lines. Biotechniques. 1998; 24(4): 624-8, 630-2).

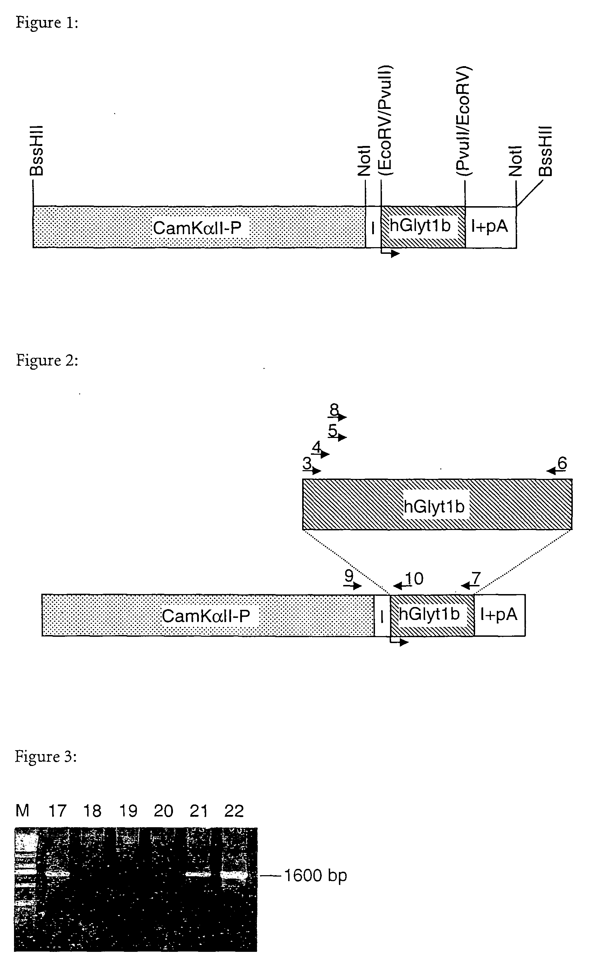

Preferably, the genetic construct is as depicted in Fig. 1.

The term "transgenic DNA" as used herein describes DNA artificially introduced and incorporated into an organism.

The term "sequence of *glyt1* gene" as used herein, describes the DNA sequence encoding GLYT1.

The present invention further provides a method of producing a non-human transgenic animal whose genome comprises transgenic DNA encoding GLYT1, comprising
a) introducing a genetic construct as described above into a non-human zygote or an non-human embryonic stem cell,
b) generating a transgenic non-human animal from said zygote or an embryonic stem cell, and thereby,
c) producing a transgenic non-human animal whose genome comprises transgenic DNA encoding GLYT1.

A further embodiment of the invention provides a method of producing a non-human transgenic animal expressing transgenic GLYT1 comprising
a) introducing a genetic construct as described above into a non-human zygote or an non-human embryonic stem cell derived from a non-human animal
b) generating a transgenic animal from said zygote or embryonic stem cell, and thereby,
c) producing a transgenic non-human animal expressing transgenic GLYT1.

Preferably, the zygote used in the methods described above is a C57BL/6J zygote. Zygotes used in the art which may also be used in the methods of this invention comprise, but not limited to, FVB/N zygotes, BALB/c zygotes, DBA/1 zygotes and DBA/2 zygotes.

Preferably, the embryonic stem cell used in the methods described above is a C57BL/6J embryonic stem cell. Stem cells used in the art which may also be used in the methods of this invention comprise, but are not limited to, BALB/c embryonic stem cells, DBA/2J embryonic stem cells, CBA/J embryonic stem cells and embryonic stem cell lines of mouse strains 129.

The zygote or embryonic stem cell may derive from any non-human animal. Preferably, the zygote or embryonic stem cell derives from a rodent. More preferably, the zygote or embryonic stem cell derives from a mouse.

The introduction of the genetic construct in the zygote may be by microinjection of the DNA. The introduction of the genetic construct in the embryonic stem cell may be by viral infection.

For example, the transgenic non-human animals of the above-described methods may be generated by culturing the zygotes after microinjection, transferring the cultured zygotes into a pseudo-pregnant non-human animal and breeding transgenic non-human animals.

The term "transgenic GLYT1" as used herein describes GLYT1 protein originating from DNA artificially introduced and incorporated into an organism.

The term "transgenic animal" as used herein describes an animal comprising transgenic DNA in their genome. This transgenic DNA may be incorporated somewhere in the genome.

The present invention further provides the transgenic non-human animal produced by any of the above described methods.

In one embodiment of the invention, transgenic non-human animals whose genome comprises transgenic DNA encoding GLYT1 are provided. In a preferred embodiment the transgenic non-human animal comprises a genetic construct as depicted in Fig. 1.

In another embodiment transgenic non-human animals expressing transgenic GLYT1 are provided. In a preferred embodiment, the transgenic GLYT1 is tissue-specific expressed, i.e., in the brain. In a more preferred embodiment, the transgenic GLYT1 is specifically expressed in the forebrain of the transgenic non-human animal. In another embodiment, the expression of the transgenic GLYT1 may be controlled, e.g., by addition of specific inducer or repressor substances.

In the described transgenic non-human animals overproducing GLYT1 protein a modulation of NMDA receptor activity is expected *in vivo* by alteration of the endogenous glycine level. Due to the lack of GLYT2 receptors in hippocampal and cerebral cortex regions, an overexpression of GLYT1 in these regions leads to decreased levels of glycine in glutamatergic synapse and thus depressing NMDA receptor function. Therefore a mutant mouse overexpressing GLYT1 is expected to develop behavioral alterations and abnormalities respectively to schizophrenia and cognitive impairments.

The transgenic non-human animal may be any non-human animal known in the art, which may be used for the methods of the invention. Preferably, the transgenic non-human animal is a mammal, more preferably the transgenic non-human animal is a rodent. Most preferably, the transgenic animal of the invention is a mouse.

The transgenic non-human animals described above may be analyzed genetically, molecularly and behaviorally.

The present invention also relates to descendants of the transgenic non-human animals as provided by the invention, obtained by breeding with the same or with another genotype.

The present invention further provides a cell line or primary cell culture, tissue as well as an organotypic brain slice culture derived from the transgenic non-human animals as provided by the invention or its descendants.

Cell culture based models can be prepared by two methods. Cell cultures can be isolated from the non-human transgenic animals or prepared from established cell cultures using the same constructs with standard cell transfection techniques.

Integration of the genetic construct comprising transgenic DNA encoding GLYT1, can be detected by various methods comprising genomic Southern blot and PCR analysis using DNA isolated from tail biopsies of two to three weeks old mice.

It will be apparent to the person skilled in the art that there are a large number of analytical procedures which may be used to detect the expression of the transgenic DNA comprising methods at the RNA level comprising mRNA quantification by reverse transcriptase polymerase chain reaction (RT-PCR) or by Northern blot, *in situ* hybridization, as well as methods at the protein level comprising histochemistry, immunoblot analysis and *in vitro* binding studies. Quantification of the expression levels of the targeted gene can moreover be determined by the ELISA technology, which is common to those knowledgeable in the art.

Quantitative measurement can be accomplished using many standard assays. For example, transcript levels can be measured using RT-PCR and hybridization methods including RNase protection, Northern blot analysis, and RNA dot analysis. Immunohistochemical staining as well as Western blot analysis can also be used to assess the presence or absence of the transgenic GLYT1 protein.

The transgenic animals of the invention may be further characterized by methods known in the art, comprising immunohistochemistry, electron microscopy, Magnetic Resonance Imaging (MRI) and by behavioral studies addressing neurological and cognitive functions. Examples of behavioral tests are: spontaneous behavior, behavior related to cognitive functions, pharmacologically-disrupted behavior, grip strength, wire manoeuvre, swim test, rotarod, locomotor activity, Morris water maze, Y-maze, lightdark preference, passive and active avoidance tests.

A further objective of the present invention is the use of the transgenic non-human animal as described, or a cell line or tissue or an organotypic brain slice culture as derived thereof, as a model for studying the ability of compounds to reduce psychotic behavior. Additionally, these transgenic animals, cells or tissue or organotypic brain slice culture as derived thereof, may be used as a model for studying the effects of depressing synaptic NMDA receptor function.

In a further embodiment, a method for evaluating the *in vivo* effects of GLYT1 function on NMDA receptor activation is provided, comprising determining NMDA receptor activity, synaptic plasticity and behavior comprising learning and memory in a transgenic non-human animal whose genome contains a transgenic sequence of *glyt1* gene in a way that active GLYT1 protein is overexpressed, and comparing the NMDA receptor activity, synaptic plasticity and behavior to those in a control.

Controls may comprise any non-human animal, wherein transgenic DNA encoding GLYT1 is not introduced in a way that active GLYT1 protein is overexpressed, or wherein the animal comprises exclusively native *glyt1* genes. Assessment of the behavior may comprise spontaneous behavior, behavior related to cognitive functions comprising spatial short- and long-term memory, object recognition memory, associative emotional memory, conditioned fear extinction, and pharmacologically-disrupted behavior comprising drug-induced hyperlocomotion, drug-induced social withdrawal, drug-induced deficits in prepulse inhibition and drug-induced memory loss.

In another embodiment, a method of testing GLYT1 inhibitor compounds for the capability to enhance the NMDA receptor function, which method comprises administering a GLYT1 inhibitor compound to a transgenic non-human animal whose genome contains a transgenic sequence of *glyt1* gene in a way that active GLYT1 protein is overexpressed, or a cell line or primary cell culture or an organotypic brain slice culture derived thereof, and determining the effect of the compound comprising assessing behavior, electrophysiology and histology, and comparing the behavior, electrophysiology and histology to those of a control.
GLYT1 inhibitor compounds which maybe used in the method of the invention are any GLYT1 inhibitor compounds known in the art comprising, but not limited to, ALX-5407(NPS Pharmaceuticals) and ORG-24598 (Organon).

Controls may comprise any animal, cell line or primary cell culture or organotypic brain slice culture or tissue, wherein transgenic DNA encoding GLYT1 is not introduced in a way that active GLYT1 protein is overexpressed, or wherein the animal, cell line or primary cell culture or organotypic brain slice culture comprises exclusively native *glyt1* genes. Assessment of the behavior may comprise spontaneous behavior, behavior related to cognitive functions comprising spatial short- and long-term memory, object recognition memory, associative emotional memory, conditioned fear extinction, and pharmacologically-disrupted behavior comprising drug-induced hyperlocomotion, drug-induced social withdrawal, drug-induced deficits in prepulse inhibition and drug-induced memory loss.

The present invention further relates to a kit for testing compounds for capability to enhance the NMDA receptor activity comprising transgenic non-human animal whose genome contains a transgenic sequence of *glyt1* gene in a way that active GLYT1 protein is overexpressed, or a cell line or primary cell culture or tissue or an organotypic brain slice culture or tissue derived thereof, and a means for determining whether a compound exhibits the capability to enhance the NMDA receptor activity.

Furthermore, the use of a transgenic non-human animal, whose genome contains a transgenic sequence of *glyt1* gene so that active GLYT1 protein is overexpressed, or a cell line or primary cell culture or tissue or an organotypic brain slice culture or tissue derived thereof is provided as model for studying the effect of compounds on the psychotic behaviour and for testing of compounds for GLYT1-specific inhibitory effects.

The invention further provides the transgenic animals, methods, compositions, kits, and uses substantially as described herein before especially with reference to the foregoing examples.

Having now generally described this invention, the same will become better understood by reference to the specific examples, which are included herein for purpose of illustration only and are not intended to be limiting unless otherwise specified, in connection with the following figures.

Figure 1 shows a schematic diagram of the genetic construct comprising the mouse CamKαII-Promoter, cDNA encoding human GLYT1b (hGlyt1b) with incorporated introns (I), wherein one intron comprises a polyadenylation site (pA). Restriction sites are given above the diagram.

Figure 2: Schematic diagram of the primer pairs used for cloning of the *glyt1b*-cDNA (SEQ. ID NOs: 3 to 8) and for the verification of recombination events (SEQ. ID NOs: 9 and 10).

Figure 3: PCR for transgenic cassette 3' of CamKaII-promoter to 5' of *glyt1b* gene (SEQ. ID NOs: 9 and 10). The amplicon resulting form the genetic construct has a size of 1600bp. The endogenous gene does not give an amplicon. 17-22: F1-mice, M: Marker.

### Examples

Commercially available reagents referred to in the examples were used according to manufacturer's instructions unless otherwise indicated.

### Example 1: Generation of mice:

### A) Cloning of human GLYT1b cDNA:

Based on sequence information from the published human glyt1b-cDNA-sequence (note: the GLYT1b -sequence is published as sequence of 1c; SEQ. ID NO: 1) primers (SEQ. ID NOs: 3 to 6)) were derived for cloning of the glyt1b-cDNA from a pACT2-cDNA library of whole human brain (Clontech) by a nested PCR. The amplified cDNA was subcloned into the NheI and EcoRI restriction-sites of the cloning vector pCI (Promega; SEQ. ID NO: 10).

### B) Cloning of hGlyt1b-transgene:

The human glyt1b cDNA was reamplified from the above vector using the primers huGlyt1b-2147FLAG-PvuII-rev (SEQ. ID NO: 7) and huGlyt1b-234c (SEQ. ID NO: 8). The amplicon was cut with PvuII, purified and cloned into the EcoRV-site of the vector pNN265 (M. Mayford, E. Kandel, Columbia University, New York, USA; Choi, T., et al., Mol Cell Biol, 1991. 11(6): p. 3070-4) for the addition of introns and a polyA-sequence resulting in the vector pNN265-hGlyt1b-FLAG. The cDNA-part was sequenced and compared to the published sequence to verify the correct amplification by the used Pwo-polymerase. No mutation was found.

To generate a forebrain-specific neuronal expression pattern of the transgene, the hGlyt1b-cDNA was subsequently cloned into a vector containing the mouse CamKαII-promoter (Mayford, M., et al., Science, 1996. 274(5293): p. 1678-83). For this, the multicloning site of a p Bluescript II SK⁺ plasmid (Stratagene) was substituted with a minimal cloning site containing the restriction-sites KpnI, HindIII and NotI, only. After that, the promoter-cassette (SEQ. ID NO: 2) was removed from the vector pNN279 (Mayford, M., et al., Science, 1996. 274(5293): p. 1678-83) by NotI and HindIII and cloned into the modified pBluescript II SK⁺ vector. The hGlyt1b-cDNA together with surrounding introns was removed from pNN265-hGlyt1b-FLAG by NotI-digest and cloned behind the mCamKαII-promoter into the unique NotI-site (Fig. 1).

### C) Generation of transgenic mice:

The transgenic cassette was excised from the vector backbone by BssHII-digest and purified. The DNA was injected into C57BL/6J zygotes (available from: The Jackson Laboratory, 600 Main Street, Bar Harbor, Maine 04609 USA) at a concentration of 3 ng/µl to generate transgenic mice according to established procedures (Hogan, B.C., F; Lacy, E, 1986, New York: Cold Spring Harbor Laboratory Press). Genomic DNA of subsequent offspring was screened by PCR with the primers pNN279-7431c (SEQ. ID NO: 9) and huGlyt1b-786nc (SEQ. ID NO: 10), which amplify a 1600 bp fragment of the transgenic cassette, for the presence of the transgene (Fig. 2 and 3). Founders identified in this screening were mated to C57BL/6J mice to establish the line.

### Example 2: Molecular analysis of GLYT1b transgenic mice

### A) Histological analysis of GLYT1b mutant mice

The overexpression of GLYT1b in the brains of mutant mice was confirmed by immunohistochemistry and by Western blot analysis using the GLYT1-specific antibodies raised in rabbits and guinea pigs.

### B) Electrophysiological analysis of GLYT1b mutant mice

NMDA receptor activation is required for induction of certain forms of long term potentiation (LTP) (Bliss, T.V. and G.L. Collingridge, Nature, 1993. 361(6407): p. 31-9). Potentiation induced by theta burst stimulation in hippocampal slices of GLYT1b-transgenics was compared with wild-type controls throughout the post-tetanus period as described previously (Kew, J.N., et al., J Neurosci, 2000. 20(11): p. 4037-49). It was determined whether GLYTlb-transgenic mice exhibit a different level of LTP-potentiation compared to wild-type controls.

### C) Preparation of forebrain and brainstem synaptosomes

Mice were sacrified and brain tissue were dissected on ice and subsequent procedures performed at 4°C. Tissues were homogenized in 10 vol (w/v) of 10 mM Tris-HCl pH 7.4 containing 0.32 M sucrose and 1 mM Pefabloc (cocktail of protease inhibitors) (buffer A) using a glass/teflon homogeniser (800 rpm 10 times). The homogenate was centrifuge 5 minutes at 1300 x g. The supernatant was carefully decanted and kept on ice, while the pellet was suspended in 5 vol (of the original weight) of buffer A, homogenised and centrifuged as described. The second supernatant was added to the first and centrifuged at 17,000 x g for 20 minutes. The resulting pellet (crude synaptosomal fraction) was suspended in 5 vol (of the original weight) of Krebs-Ringer solution, pH 7.4 containing 10 mM glucose (KRB).

### D) Glycine uptake

The assays were performed in 96-well plates. Aliquots of mice forebrain (0.1 mg) and brainstem (0.05 mg) synaptosomal preparations were incubated at 22°C in KRB together with 120 nM [3H] glycine in a total volume of 250 µl for 30 minutes. Incubation was stopped by rapid filtration onto 96 well Packard GF/B unifilter plates, followed by 3 washes with ice-cold KRB. After addition of scintillation solution the radioactivity content of the wells was measured.

### E) Saturation binding for MK801

The assays were performed in 96-well deep plates. Aliquots of mice forebrain and brainstem synaptosomal preparations (0.07 mg) were incubated at 22°C in 20 mM Hepes-KOH, pH 7.4 containing 100 µM glutamate and 30µM glycine together with increasing concentration(0.03nM- 300 nM) of [3H] MK801 in a total volume of 0.5 ml for 1 hour. Non-specific binding was defined with 10 µM MK801. Incubation was stopped by rapid filtration onto 96 well Packard GF/C unifilter plates, followed by 3 washes with ice-cold 20 mM of Hepes-KOH, pH 7.4. After addition of scintillation solution the radioactivity content of the wells was measured.

### F) Extracellular glycine levels in vivo

To assess the impact of increased GLYT1b expression on the extracellular level of glycine a microdialysis study was performed. Adult wild-type and mutant mice were anesthetized with isoflurane and a microdialysis vertical probe (CMA7 4/2, cuprophane - membrane custome made) was inserted in the striatum (CPu, bregma A: +0.9; L: -1.8; V: -4.6). The animals were allowed to recover for three to four days before the experiment. Dialysate glycine levels were quantified according to the method of Smith and Sharp with minor modification).

### Example 3: Behavioral analysis of GLYT1 mutant mice

### A) Neurological assessment

Neurological assessment includes a number of neurological tests like flexion reflex, grip strength (g) and time (sec) spent on a rotarod at 16 and 32 rpm and body weight.

### B) Spontaneous behavior

The GLYT1b transgenic mice were observed for signs of natural exploratory behavior including body posture, gait and sensory responses (Irwin, S., Psychopharmacologia, 1968. 13(3): p. 222-57). In addition, their spontaneous locomotor activity was analyzed (activity box). Moreover, the state of anxiety was assessed by exposing to the animals to naturally aversive stimuli (elevated plus maze test and the light/dark choice test).

### C) Auditory startle and prepulse inhibition of the acoustic startle reflex (behavior related to schizophrenia)

Testing was conducted in eight startle devices each consisting of a Plexiglas cylinder (5 cm in diameter) mounted on a Plexiglas platform in a ventilated sound attenuated cubicle with a high-frequency loudspeaker producing all acoustic stimuli. The background noise of each chamber was 68dB. Movements within the cylinder were detected and transduced by a piezoelectric accelerometer attached to the Plexiglas base and digitized and stored by a computer. Beginning at the stimulus onset, 65 x 1 msec readings were recorded to obtain the animal's startle amplitude.

Each section was initiated with a 5 min acclimation period followed by five successive 110 dB trials, which were not included in the analysis. Ten different trial types were then presented: startle pulse alone (ST110, 110 dB/40 msec); eight different prepulse trials in which either 20-msec-long 72, 78, 84, and 90 dB stimuli were presented alone (P72, P78, P84, P90) or preceded the 110 dB pulse by 100 msec (PP72, PP78, PP84, PP90); and finally one trial in which only the background noise was presented (NST) to measure the baseline movements in the cylinder. All trials were presented in a pseudorandom order, and the average intertrial interval (ITI) was 15 msec. The startle data and percentage prepulse inhibition (PPI) were analyzed by two-ways ANOVA.

### D) Nest building

To quantify the ability of mutant mice to make nests by shredding a tissue, folded pieces of tissue paper were placed into each cage, and 24 hr later the nests were assessed.

### E) Intracerebroventricular NMDA-induce convulsions.

Seizures were induced by injection of NMDA (5nM in 1 µl) into the lateral ventricle of conscious mice. Immediately after injection, animals were placed in Plexiglas boxes and observed for a period of 5 minutes. The latency (in seconds) for each mouse to exhibit wild running phase and clonic convulsions was recorded for mutant mice versus wild-type mice.

### F) Behavior related to cognitive functions

### Spatial short- and long-term memory

The delayed matching spatial working memory task was performed as described by Durkin (Durkin, T.P., et al., Behav Brain Res, 2000. 116(1): p. 39-53). Working memory was evaluated on the basis of the acquisition of a delayed matching rule in a 5-arm maze. The basic learning task was comprised of two phases. Each trial begins with a presentation phase during which the animal was exposed to a forced and rewarded visit to one arm chosen quasi-randomly, the other four arms being closed. Once rewarded, the animal was placed in a waiting cage. Following a retention interval of variable duration (2-sec, 20-sec and 40-sec delay for working memory; 5-min, 1-hr, 4-hr, 24-hr delay for short- and long-term memory), the retrieval test phase was performed during which the animal was exposed to a situation of choice among the five open arms. A correct choice of the previously visited arm was rewarded. The working memory retention capacity was expressed as a function of the retention interval by the mean percent of correct accuracy choices during successive trials with a fixed intertrial interval of 10 sec. The memory task was monitored by an automated video-tracking system.

Alternatively spatial learning and memory was assessed in the water maze paradigm described by Morris (Morris, R.G., et al., Nature, 1982. 297(5868): p. 681-3; Morris, R.G., et al., Nature, 1986. 319(6056): p. 774-6). Mice were placed in a circular pool (diameter 120 cm, height 30 cm) in which they learn to escape from milky water (20 cm depth, 20 ± 1°C) by locating a hidden platform. This target platform (7 cm diameter, 1 cm below the water surface) was located in the center of a particular quadrant of the pool, and external visual cues were positioned around the pool to facilitate navigation of the animals. During a 4 d test period, mice were placed in the water facing the wall of the pool in one of four fixed starting positions chosen randomly (3 trials per session, 3 sessions per day). The time the mouse needs to locate the target (escape latency) and the swim path and swim speed were measured using an automated video motility system. If an animal fails to find the target within 60 seconds, it was placed on the platform by hand and was allowed to remain there for an intertrial interval (10 to 20 sec). The interval between each session was 1.5 to 2 hr. After the final trial on day 4, the platform was removed, and the mice were allowed to swim freely for 60 sec. The time the mice spend in each quadrant and their swim path were recorded.

### Associative emotional memory

Associative emotional memory, which was NMDA receptor dependent, was assessed in two behavioral tasks:
i. Fear potentiated startle response: Mice were conditioned to respond to a light (CS) which was paired with a footshock (aversive US). After a delay of 24 hours, emotional memory was evaluated by the amplitude of the startle reflex elicited by different acoustic stimuli (90-110 dB) with or without the conditioned light stimulus. The presentation of the conditioned light stimulus leads to a potentiation of the acoustic startle reflex (Davis, M., Psychopharmacology (Berl), 1979. 62(1): p. 1-7).
ii. Contextual fear conditioning: Contextual fear conditioning was an implicit aversive associative learning process by which an initially neutral context acquires aversive properties after its repetitive association with an unconditioned aversive stimulus (US). The animals were exposed to a new chamber where they were treated after few minutes to successive electric foot shocks (US) that elicit unconditioned fear responses (freezing behavior) (Phillips, R.G. and J.E. LeDoux, Behav Neurosci, 1992. 106(2): p. 274-85). Contextual fear conditioning was measured by the amount of freezing in response to re-exposure to the context. The conditioned freezing response was tested at different periods of time after training in order to evaluate short-term (1 to 3 hrs) and long-term (1 to 10 days) contextual memory.

### Conditioned fear extinction

Extinction of a learned fear response represents a form of behavioral plasticity that was thought to rely on the formation of a new form of memory rather than an erasure of the original learned association (Falls, W.A., M.J. Miserendino, and M. Davis, J Neurosci, 1992. 12(3): p. 854-63). Recently it has been shown that conditioned fear extinction involves an NMDA receptor-dependent process (Tang, Y.P., et al., Nature, 1999. 401(6748): p. 63-9). Following a delay of 24-hr after training, extinction of conditioned freezing can be evaluated by the time-dependent decrease of the amount of freezing in response to repetitive exposure to the context during five consecutive days.

### G) Pharmacologically disrupted behavior

A distinct range of schizophrenia-type symptoms, including hyperlocomotion, deficits in prepulse inhibition (PPI) and memory deficit can be induced by the administration of apomorphine, D-amphetamine or the non-competitive NMDA receptor antagonist PCP. The minimally effective dose in disrupting behavior in wildtype mice was used. It was then tested whether GLYT1b transgenic mice displayed a different susceptibility to the pharmacological disruption of behavior.

### Drug-induced hyperlocomotion

In the open field (activity box) the effect of D-amphetamine on locomotor activity and stereotypic behavior was assessed by recording the horizontal locomotor activity, vertical activity, stereotypic movements and the time spent in the center of the open field.

### Drug-induced social withdrawal

To assess the effect of GLYT1b overexpression on the behavioral response to D-amphetamine or PCP on social behavior a social exploration test was used (Crestani, F., F. Seguy, and R. Dantzer, Brain Res, 1991. 542(2): p. 330-5). An individually housed male mouse was exposed for 5 minutes to a juvenile female mouse. Social interactions including ano-genital and neck sniffing, heterogrooming and pursuits were recorded via a video tracking system before and after drug administration. The drug-induced social exploration deterioration was examined at different time intervals (30 min, 2 hours, 4 hours and 24 hours) after drug administration.

### Drug-induced deficits in prepulse inhibition

Disruption of prepulse inhibition (PPI) by apomorphine or PCP was a frequently used model of the sensorimotor gating deficits (Kretschmer, B.D., et al., Eur J Pharmacol, 1997. 331(2-3): p. 109-16; Bakshi, V.P. and M.A. Geyer, J Neurosci, 1998. 18(20): p. 8394-401; Bakshi, V.P., et al., J Pharmacol Exp Ther, 1999. 288(2): p. 643-52) by which attention and sensorimotor gating can be evaluated. PPI was the attenuation of an acoustic or tactile startle response following the presentation of a non-startle-inducing prepulse stimulus. The animal's startle response following acoustic stimuli was assessed.

### Drug-induced memory loss

PCP- or apomorphine-induced impairment of memory functions was assessed in the delayed matching spatial memory task described above.

### H) Reversal of in vitro and in vivo deficits/impairments by specific GLYT1 inhibitors in GLYT1b mutant mice.

It was determined whether specific GLYT1 inhibitors were able to reverse impairments/deficit observed in vitro (see example 2 paragraphs B, D and E) and in vivo (see Example 2 paragraph F, Example 3, paragraphs A to F).

### I) Reversal of in vitro and in vivo deficits/impairments by specific GLYT1 inhibitors in pharmacologically challenged GLYT1b mutant mice.

It was determined whether specific GLYT1 inhibitors were able to reverse, in GLYT1b mutant mice, their altered sensitivity to pharmacological disruption of behavior (see example 3 paragraph G).

## Claims

1. A genetic construct comprising a DNA sequence encoding GLYT1 operatively linked to a promoter.

2. A genetic construct according to claim 1 wherein the DNA sequence encodes an isoform of GLYT1.

3. A genetic construct according to claim 1, wherein the DNA sequence encodes GLYT1b.

4. A genetic construct according to any one of claims 1 to 3, wherein the coding sequence is a human sequence.

5. A genetic construct according to any one of the claims 1 to 4, wherein the promoter is a tissue-specific promoter.

6. A genetic construct according to any one of the claims 1 to 4 wherein the promoter is a forebrain-specific promoter.

7. A genetic construct according to any one of the claims 1 to 4, wherein the promoter is a controllable promoter.

8. A method of producing a transgenic non-human animal whose genome comprise transgenic DNA encoding GLYT1, comprising
a) introducing a genetic construct according to any one of the claims 1 to 7 into a non-human zygote or an non-human embryonic stem cell,
b) generating a transgenic non-human animal from said zygote or embryonic stem cell, and thereby,
c) producing a transgenic non-human animal whose genome comprise transgenic DNA encoding GLYT1.

9. A method of producing a non-human transgenic animal expressing transgenic GLYT1 comprising
a) introducing a genetic construct according to any one of the claims 1 to 7 into a non-human zygote or an non-human embryonic stem cell,
b) generating a transgenic non-human animal from said zygote or embryonic stem cell, and thereby,
c) producing a transgenic non-human animal expressing transgenic GLYT1.

10. A transgenic non-human animal produced by the method according to any one of the claims 8 to 9.

11. A transgenic non-human animal, whose genome comprises a genetic construct according to any of the claims 1 to 7.

12. A transgenic non-human animal according to claim 10 or 11 wherein the transgenic animal is a rodent.

13. A transgenic non-human animal according to claim 10 or 11 wherein the transgenic animal is a mouse.

14. A transgenic non-human animal according to any one of the claims 10 to 13 overexpressing GLYT1 protein.

15. A transgenic non-human animal according to any one of the claims 10 to 13 overexpressing GLYT1 protein tissue-specific.

16. Descendants of the transgenic non-human animal according to any of the claims 10 to 15.

17. A cell line or primary cell culture derived from a transgenic non-human animal or its descendants according to any one of claims 10 to 16.

18. A tissue or an organotypic brain slice culture derived from a transgenic non-human animal or its descendants according to any one of the claims 10 to 16.

19. Use of a transgenic non-human animal according to claims 10 to 16, or a cell line or primary cell culture according to claim 17, or a tissue or an organotypic brain slice culture according to claim 18 as a model for studying the effect of compounds on the psychotic behaviour.

20. Use of a transgenic non-human animal according to claims 10 to 16, or a cell line or primary cell culture according to claim 17, or a tissue or an organotypic brain slice culture according to claim 18 for testing compounds for GLYT1-specific inhibitory effects.

21. Use of a transgenic non-human animal according to claims 10 to 16, or a cell line or primary cell culture according to claim 17, or a tissue or an organotypic brain slice culture according to claim 18 as a model for studying the effect of depressing synaptic NMDA receptor function.

22. A method for evaluating the *in vivo* effects of GLYT1 function on NMDA receptor activation comprising determining NMDA receptor activity, synaptic plasticity and behavior comprising learning and memory in a transgenic non-human animal according to any one of claims 10 to 16, and comparing the NMDA receptor activity, synaptic plasticity and behavior to those in a control.

23. A method of testing GLYT1 inhibitor compounds for capability to enhance NMDA receptor activity which method comprises administering a GLYT1 inhibitor compound to a transgenic non-human animal according to claims 10 to 16, or a cell line or primary cell culture according to claim 17, or a tissue or an organotypic brain slice culture according to claim 18, and determining the effect of the compound comprising assessing behavior, electrophysiology and histology, and comparing the behavior, electrophysiology and histology to those of a control.

24. A kit for testing GLYT1 inhibitor compounds for capability to enhance the NMDA receptor activity comprising a transgenic non-human animal according to any one of claims 10 to 16, or a cell line or primary cell culture according to claim 17, or tissue or an organotypic brain slice culture or tissue according to claim 18, and a means for determining whether a compound exhibits the capability to enhance the NMDA receptor activity.

25. The genetic constructs, methods, transgenic animals, kits and uses substantially as described herein before especially with reference to the foregoing Examples.


SEQUENCE LISTING

<110> F. Hoffman-La Roche

<120> Glyt1 transgenic mouse

<130> 22511

<160> 11

<170> PatentIn version 3.3

<210> 1
<211> 2202
<212> DNA
<213> Homo sapiens

<220>
<221> glycine transporter type 1c (alternatively spliced)
<222> (1)..(2202)
<223> representative cDNA (S70612, as of 18.02,2004),

for gene under UniGene ID Hs.442590

<400> 1
gcccacacac cccactccag ctccggagca cccgtgctgg gctgcatggg gactggccgg 60

aggggcaggg ccaggggagc gggtaggcag agcttcggga ggagatgagg tgaaagtaat 120

tgacgctgcc cagcccggca gtgggagagg caggggatgc gtcagtgtcg cgctggagct 180

ggcagaggtg atgagcggcg gagacacgcg gggctgcgat cgctcgcccc aggatggccg 240

cggctcatgg acctgtggcc ccctcttccc cagaacagaa tggtgctgtg cccagcgagg 300

ccaccaagag ggaccagaac ctcaaacggg gcaactgggg caaccagatc gagtttgtac 360

tgacgagcgt gggctatgcc gtgggcctgg gcaatgtctg gcgcttccca tacctctgct 420

atcgcaacgg gggaggcgcc ttcatgttcc cctacttcat catgctcatc ttctgcggga 480

tccccctctt cttcatggag ctctccttcg gccagtttgc aagccagggg tgcctggggg 540

tctggaggat cagccccatg ttcaaaggag tgggctatgg tatgatggtg gtgtccacct 600

acatcggcat ctactacaat gtggtcatct gcatcgcctt ctactacttc ttctcgtcca 660

tgacgcacgt gctgccctgg gcctactgca ataacccctg gaacacgcat gactgcgccg 720

gtgtactgga cgcctccaac ctcaccaatg gctctcggcc agccgccttg cccagcaacc 780

tctcccacct gctcaaccac agcctccaga ggaccagccc cagcgaggag tactggaggc 840

tgtacgtgct gaagctgtca gatgacattg ggaactttgg ggaggtgcgg ctgcccctcc 900

ttggctgcct cggtgtctcc tggttggtcg tcttcctctg cctcatccga ggggtcaagt 960

cttcagggaa agtggtgtac ttcacggcca cgttccccta cgtggtgctg accattctgt 1020

ttgtccgcgg agtgaccctg gagggagcct ttgacggcat catgtactac ctaaccccgc 1080

agtgggacaa gatcctggag gccaaggtgt ggggtgatgc tgcctcccag atcttctact 1140

cactggcgtg cgcgtgggga ggcctcatca ccatggcttc ctacaacaag ttccacaata 1200

actgttaccg ggacagtgtc atcatcagca tcaccaactg tgccaccagc gtctatgctg 1260

gcttcgtcat cttctccatc ctcggcttca tggccaatca cctgggcgtg gatgtgtccc 1320

gtgtggcaga ccacggccct ggcctggcct tcgtggctta ccccgaggcc ctcacactac 1380

ttcccatctc cccgctgtgg tctctgctct tcttcttcat gcttatcctg ctggggctgg 1440

gcactcagtt ctgcctcctg gagacgctgg tcacagccat tgtggatgag gtggggaatg 1500

agtggatcct gcagaaaaag acctatgtga ccttgggcgt ggctgtggct ggcttcctgc 1560

tgggcatccc cctcaccagc caggcaggca tctattggct gctgctgatg gacaactatg 1620

cggccagctt ctccttggtg gtcatctcct gcatcatgtg tgtggccatc atgtacatct 1680

acgggcaccg gaactacttc caggacatcc agatgatgct gggattccca ccacccctct 1740

tctttcagat ctgctggcgc ttcgtctctc ccgccatcat cttctttatt ctagttttca 1800

ctgtgatcca gtaccagccg atcacctaca accactacca gtacccaggc tgggccgtgg 1860

ccattggctt cctcatggct ctgtcctccg tcctctgcat ccccctctac gccatgttcc 1920

ggctctgccg cacagacggg gacaccctcc tccagcgttt gaaaaatgcc acaaagccaa 1980

gcagagactg gggccctgcc ctcctggagc accggacagg gcgctacgcc cccaccatag 2040

ccccctctcc tgaggacggc ttcgaggtcc agtcactgca cccggacaag gcgcagatcc 2100

ccattgtggg cagtaatggc tccagccgcc tccaggactc ccggatatag cacagctgcc 2160

aggggagtgc caccccaccc gtgctccacg agagactgtg ag 2202

<210> 2
<211> 7988
<212> DNA
<213> Mus musculus

<220>
<221> Mouse calcium/calmodulin-dependent protein kinase II alpha
subunit gene 5' flanking sequences
<222> (1)..(7988)
<223> representative cDNA (AJ222796, as of 18.02.2004)

<400> 2
cgatcttttt tccgtaaact caataccagg ctgatgtccc accggatctg atggcttagg 60

gtggcaggga atctcagttc ccctcagaca ctctcccttt gctggttctc agggaggagg 120

caaggtcaag tcttcatctg taggcacgtg gagggagggc acagaagccc tcagctgaat 180

agggtgggac ttggggaagg gcagcaacca ggctgggttg cctgggtcac aatcctgcct 240

ctttcctgat gagtttcctt tttgccctca ggttacctat agcagcattc tgcctcaatc 300

tcacccctaa gatgagctct ggtgacttta ggactccagt gtacacatgt gtctggggcc 360

atggcagggt ttcttgctga ccttgtcacc ttccagacaa cttgagtcca tgaccctctt 420

tccagctctc tgtggtgctc ttggatatca gctggagtat ggccagctgg ctgctgctct 480

gttgaacaac tcaatgagag aacggacagg gtaggctctg agaaatcttt acgttcctgg 540

agcctcatga cttgggagcc tagtggaatt cttctctttt ggtccccaac atctgggggg 600

agggggaact ggctgagcct gagccactgt atagtgaggg tgggggaaac agctgtgaaa 660

ggagcctttg atttggtctt gaacacagtt cttccccaca gggccttgat ttccctactt 720

gcaaaggagt agggaaatat gagccttggc tctgcctacc tcacgttgct ggtgctgtag 780

aaaactggcc aggctgatgg ttgtggagga gcctgtgaac ttgattaaag tgccattatc 840

cagaggcaag agatgctggt ctgtgtgtgt gtgagtgtgt gtgtgtgtgt gtgtgtgtgt 900

gtgtgtgtgt cttggagacc tgtgtgagct cgatgctagc agaggggaca gaaggtaggt 960

gggaaggaat gaaggaatga aggaaggaaa gaaggaaggt tgaaaggaag aaaggaagga 1020

aggaaggaag gaaggaagga aggaaggaag gaaggaagga aggaaggaag gtcctgccac 1080

aggcttacca tgtagctgca ggcaaacccc tgaccctctc tgggcctaag tgtttctcta 1140

cacacaatgg atgattcaag agtccttact tttggtggtt acaggcaccc ctgtgcacat 1200

ttgcatctgg ggtggggggg acacaggctt ggtagtgttg aggagggggg tggttgtaga 1260

gcctgctagc tgcacactgc gttctgcata tctcccttca ggtcccagtc ggcgcagtgt 1320

gtgtaggcga aggccctgct gtgaattttg aaaatagtta tttttgtcac tggcaaagga 1380

ggccctgtta ggactcgtca gcttgtggat gagcgggatg ggtggagtgg ggtgggtgcg 1440

gtgccgccgt ggggggttac cgctgcttgc agggctgcat cgcccaggca gtgactgaat 1500

cctgcatgag ggcctggcct aggctgtggg gaggagatga ccactgcgtc ctagatcttt 1560

ccttagccct gtgcttcctt tcttcctttt ttccttaaga tttatttcta atgatgcgta 1620

ggttgtgtat ttgtgtgtgg gtatgtgcac ttgaataaag gacccacaga ggctatagac 1680
atcagatcct cctagagctg gggttacaga gggcgtgagt tgtccaacat gggcaccgga 1740
aaataaactt atgtgctcta cacgaccgag ctgtctctcc aacaccagcc ctcttctttt 1800
gacttttctc atctccctca cttgtatgtt ttcccttcct cgataatgct gatacccaga 1860
tggtaggcac ggcccaggat aggcaggggt ctctgcgctc cagtggctgt agtggttttc 1920
ctgcctgctc tgaagaagac actggctaag gtggtgtctt agcctactgt atcctagagg 1980
tgggttattc atagtctgcc cactgcccaa cacactctag gcctgctggg gcctattcta 2040
actctgcttg ctggcttgcc accctggcac acagtgtagg cttccctgta gagccaggct 2100
ttagagaact gtatgagtac ttctctgaga actgctggag gggccctgcc tgccaggact 2160
tttcaacttc cagccctgtc atctatatca cttctgagga ccccgtgtgg gggtcacgag 2220
aacaagacca tatgtagtgc tttctgttct cccttgggtc ccaggctctg aatcaatctg 2280
gtcccaagat ataagggatg attggtgctg aggctggtgt ctgtttctga agttttgaag 2340
acaagggttg gctcaagcct ccctgtgttc agtcctccac tcaatgcaga actcagtgaa 2400
ctcagaattc tcagcccaga tgccagcata gcccagcata gcccagcata gcccaggagc 2460
tactggagca tcagtttgaa accaggtccg caggaactag tgggcaacag tgtgtgaggc 2520
cagtggtctt tggggtattg tattgaattg agaggtcctg cttagcagtc agcatgccca 2580
caacctgttc tctacggtgg tccggattcc cctcagcaag cacacctgaa tctttactac 2640
atcccagttc ctggttggct cctgacttcg ggttactatg gctgtgatga aacactacga 2700
ctaaaagcaa tgtggggaag aaagttaatt ttttcactcg accttccata gacagggttc 2760
atcactaaaa gcagagggca gaggaaaaga tagctcagcg gttaagagtg ctgtctactc 2820
aacaaagagg tcttgagttc aattcccagc aaccacatgg tggctcacaa ctgtctatcc 2880
tgggatctga tgcccttttc tggcatacag gtatacatac agatagagga ctcatataca 2940
taaaataaat aaataaatct ttaaaagcaa caagggcaga aattcaagca gggcagggac 3000
ccagaggcca aagctgacgc agaggccatg gaggggtgtt gcttactggc ttgctcctca 3060
tggcttgctc agcctgtttt cttatagaac ccacaaccac caggccagag atgggaccac 3120
ccacaaaggg cagagccttt ccctatcaat cactaatggg aaaacatcct gcagtcggat 3180
cttatgaaga cattttctca gctgagcttc cctcctatca gataactcta gcttgtgtca 3240
aggtgactta aaactagcca gcacagcacc ttatgctcac atcacctggg tccctttgga 3300
gaggacatag ttaaagggag cccagaggca gtccctaggc cacaggtctt cattgcccct 3360
ctctgggacg gattagacag gctgcagacc tgttagctgg aagagttaga ttcaggcaag 3420
agcttgaatc tttacctgat cctggctatg gagtcctggc ctctaatgat cagctcccta 3480
acaacccaat ggagccatat acctgcctgg gccacggctg tgtctcctct tctttcagac 3540
actcctggct tgcctaggac acaggctagc atcctgtcaa tgccaggaag gggcacagca 3600
gggaaagagc aatgctgttg gcctgactgc catcaactgg tgtacctgtt agagggcaac 3660
ctctattctc tgcaccttgg ttcctagctc taagggatat gtggccccta aaggtcttca 3720
tagcttgata tgggaggcag gggggctaag aacagcgcaa gagtggtgag cttgcacaga 3780
cccggatttg atctctgggt gagtgaggag gaaatgagat gggggtgggg gaagccctat 3840
ttctagctgt cttagcatag gaactgaacc tccttctgca gggcctgtgt cactgcccct 3900
ttcccccagg gagggcccct gcacggggca cctcagggca cagccctttt tccctccctc 3960
ctctcttaga cctggaatta ctcaacatcc tgccctgact cagttgctct cccctcagac 4020
cctcacagtc ttccttctct tctggcccac ttttggctga gcctgccccc aactttttct 4080
gcccttagtg ggacaggccc catggggacc attcagatgg cacttttttc cccccctggg 4140

gtggttttct gtggtggtgc cctattcagg caactgcaag accctgtggc atttagcata 4200
tgcatgagag cacatgaaga agctagctat ccctgtgtgc tgaggattgt aatcctctct 4260
catccttccc ttgtctcctg gaacccagtc cagcctcctg tccctcccgt tgacacgagc 4320
caatgctggc tcagcaaact ccagggctcc cacccctggc catcagccct tggcacacag 4380
gcttgtgctt gagtactgca cacgtgttgc agctggggta cacgtgctgg actgttatgc 4440
ctactgtggc cccgggggtg tgtgggaagt ctggcagaac caatccctcc atcccccgat 4500
gcaatcatca gcttattctc tcacggccac tcgggcatgc ttgactcctt gatgcccgcc 4560
gccactaggc acagctgcca gctttgtggg cacagaggat gtggcgaatt agtggtcatg 4620
cctcctcagt ggaatggcaa ttgcactcag catgcaggtg tctaccaaag gcagtcccta 4680
catccccgat gtactctcga gacccatcta aggactagat ctagtctcta gaaggtccca 4740
tgcagatgta agacagccct ccacagggag attcttccag ctagttctct attatcagat 4800
gggtctaaga tcctaggacc tgcctatccc ttagccctgc attcagcgag agaaggggta 4860
aagatgtgag gatgccaggg aggaaggaaa agggcacaag gaagaaagaa agggaaggaa 4920
gctggaagca tggaaggaca aagatggtga ccacagtaga attaggatcc catggttcct 4980
gtcagtggct tcctgtgcct tcctgtgcct ccctgagccc ctggggcatc ttctaaatgc 5040
tttgctggcc tctgagccaa gcactgcata ccatcccgtg gggagtgaca ggccagcact 5100
ggtcaacgag gatgatggct acttttgttc acagggtaac atctccatgg ttacagcctt 5160
tgcacattcc tcttagtact ttaccaatct caaagcagtt gccaagccct tgggccctaa 5220
taagtgaggg tcccagtgcc ctctttttta aattccttgc catttgtttt gcagaattta 5280
ctgcaaataa agccaacccc aggcaatgtc taaaccatga gttaaccccc cagcaaggtc 5340
tcagagaact gtgccccaga gagctgccaa ggttcaggga ggagtatgag gagacaggat 5400
ttctagttcc ttaataattc cttctgtctc agccactgtg ttcatcttgt ttcagccaca 5460
aaactacctt tattggtaag gaacattatt tacccagttt cacacttgaa gaggtccaga 5520
gacgttaaca catcgattca aaagcacagc ctgtaagtca catagccact gttagctgat 5580
cgacactatt tcccctgggc aatggctggg tgattccagg gatccccttg ggaacaggct 5640
agagcactgg ctctcaacct gtgcgggtcg tgacctcctt gaggggtagg ggtgagggca 5700
gtgtcaaaca acccttttac aggagtcgtt taagaccgtt gggaaaaaaa ccagatattt 5760
gcattatttt tcgtaacaga agcaagatta tagttatgga gtagtgacaa aaattatgtt 5820
acagttggag gtcagcacag catgaggaac tgtatttaag ggttgcggca ttaggaaggt 5880
tgagaatcac tggcctagcg gatctgaatc aggaacacgg acgtacagct ctgcgccact 5940
cctgccttcc tctggtgcct ctagccttgc ccatggtgtt ctgggcctgc ctgctaccca 6000
ccagctgtgc ggccctgtga gcacaggcct ttctgctccg ctctgaattg ccacgttggc 6060
ggcagaagcg ggaagcgtat tgtgcgcaga aacaaaacgg agtggttttt tttttccttt 6120
ttctgaaggt ggtaatggtg caattagtgg cgaagccatc accccctcct ccccggctcg 6180
cctccctcct tcctctccac ctcccttctc tttctttcct gagaaaaaaa gtggctgagt 6240
tgaaaagatc tcccgtcaat ctttctgtaa cggactcagg aagagggata gagggccccc 6300
taatgtttcc agggtcctcg agcctcagtt gggtcaggca cttgttggtg ctggagaata 6360
ttcaaaggta ccactatgtt ccccacaagg gagttgagca atggattctg aggagcaagt 6420
ttgaaacaga gaatttgcgt tcccaggtct tgtgatctgc cccttgttca ctgggggaca 6480
aatgctggca tgagaccctg agacctctgc tcagccacct ttctctctct ctctctcttt 6540
ctctctctct ctctctctct ctctctctct ctctctctct ctctctgtcc ttaatggagg 6600
tgtgtgtgtg ttcaagacca agctgcagtg ttggagtgct tgtgggctca ttttaaaact 6660

tccatgtttt gccttctaga aactgaaaca taagaacccc attatggcct taggtcactt 6720
catctccatg gggttcttct tctgattttc tagaaaatga gatgggggtg cagagagctt 6780
cctcagtgac ctgcccaggg tcacatcaga aatgtcagag ctagaacttg aactcagatt 6840
actaatctta aattccatgc cttgggggca tgcaagtacg atatacagaa ggagtgaact 6900
cattagggca gatgaccaat gagtttagga aagaagagtc cagggcaggg tacatctaca 6960
ccacccgccc agccctgggt gagtccagcc acgttcacct cattatagtt gcctctctcc 7020
agtcctacct tgacgggaag cacaagcaga aactgggaca ggagccccag gagaccaaat 7080
cttcatggtc cctctgggag gatgggtggg gagagctgtg gcagaggcct caggaggggc 7140
cctgctgctc agtggtgaca gataggggtg agaaagcaga cagagtcatt ccgtcagcat 7200
tctgggtctg tttggtactt cttctcacgc taaggtggcg gtgtgatatg cacaatggct 7260
aaaaagcagg gagagctgga aagaaacaag gacagagaca gaggccaagt caaccagacc 7320
aattcccaga ggaagcaaag aaaccattac agagactaca agggggaagg gaaggagaga 7380
tgaattagct tcccctgtaa accttagaac ccagctgttg ccagggcaac ggggcaatac 7440
ctgtctcttc agaggagatg aagttgccag ggtaactaca tcctgtcttt ctcaaggacc 7500
atcccagaat gtggcaccca ctagccgtta ccatagcaac tgcctctttg ccccacttaa 7560
tcccatcccg tctgttaaaa gggccctata gttggaggtg ggggaggtag gaagagcgat 7620
gatcacttgt ggactaagtt tgttcgcatc cccttctcca accccctcag tacatcaccc 7680
tgggggaaca gggtccactt gctcctgggc ccacacagtc ctgcagtatt gtgtatataa 7740
ggccagggca aagaggagca ggttttaaag tgaaaggcag gcaggtgttg gggaggcagt 7800
taccggggca acgggaacag ggcgtttcgg aggtggttgc catggggacc tggatgctga 7860
cgaaggctcg cgaggctgtg agcagccaca gtgccctgct cagaagcccc aagctcgtca 7920
gtcaagccgg ttctccgttt gcactcagga gcacgggcag gcgagtggcc cctagttctg 7980
ggggcagc 7988

<210> 3
<211> 21
<212> DNA
<213> Homo sapiens

<220>
<221> primer
<222> (1)..(21)

<400> 3
agagcttcgg gaggagatga g 21

<210> 4
<211> 21
<212> DNA
<213> Homo sapiens

<220>
<221> primer
<222> (1)..(21)

<400> 4
agagcttcgg gaggagatga g 21

<210> 5
<211> 39
<212> DNA
<213> Homo sapiens

<220>
<221> primer
<222> (1)..(39)

<400> 5
gatctgctag ccaccatggc cgcggctcat ggacctgtg 39

<210> 6
<211> 32
<212> DNA
<213> Homo sapiens

<220>
<221> primer
<222> (1)..(32)

<400> 6
gatcggaatt cctatatccg ggagtcctgg ag 32

<210> 7
<211> 72
<212> DNA
<213> Homo sapiens

<220>
<221> primer
<222> (1)..(72)

<400> 7
gatccagctg gctagatatc ctacttgtca tcgtcgtcct tgtaatcgat atctatccgg 60

gagtcctgga gg 72

<210> 8
<211> 38
<212> DNA
<213> Homo sapiens

<220>
<221> primer
<222> (1)..(38)

<400> 8
gatccagctg caccatggcc gcggctcatg gacctgtc 38

<210> 9
<211> 20
<212> DNA
<213> Mus musculus

<220>
<221> primer
<222> (1)..(20)

<400> 9
cagctgttgc cagggcaacg 20

<210> 10
<211> 21
<212> DNA
<213> Homo sapiens

<220>
<221> primer
<222> (1)..(21)

<400> 10
gaggctgtgg ttgagcaggt g 21

<210> 11
<211> 4006
<212> DNA
<213> Artificial

<220>
<223> Description of artificial seqüence: mammalian expression vector

<220>
<221>  cloning vector pCI
<222>  (1)..(4006)
<223>  DNA sequence (U47119 as of 18.02.2004)

<400>  11
tcaatattgg ccattagcca tattattcat tggttatata gcataaatca atattggcta    60

ttggccattg catacgttgt atctatatca taatatgtac atttatattg gctcatgtcc   120

aatatgaccg ccatgttggc attgattatt gactagttat taatagtaat caattacggg   180

gtcattagtt catagcccat atatggagtt ccgcgttaca taacttacgg taaatggccc   240

gcctggctga ccgcccaacg acccccgccc attgacgtca ataatgacgt atgttcccat   300

agtaacgcca atagggactt tccattgacg tcaatgggtg gagtatttac ggtaaactgc   360

ccacttggca gtacatcaag tgtatcatat gccaagtccg ccccctattg acgtcaatga   420

cggtaaatgg cccgcctggc attatgccca gtacatgacc ttacgggact ttcctacttg   480

gcagtacatc tacgtattag tcatcgctat taccatggtg atgcggtttt ggcagtacac   540

caatgggcgt ggatagcggt ttgactcacg gggatttcca agtctccacc ccattgacgt   600

caatgggagt ttgttttggc accaaaatca acgggacttt ccaaaatgtc gtaataaccc   660

cgccccgttg acgcaaatgg gcggtaggcg tgtacggtgg gaggtctata taagcagagc   720

tcgtttagtg aaccgtcaga tcactagaag ctttattgcg gtagtttatc acagttaaat   780

tgctaacgca gtcagtgctt ctgacacaac agtctcgaac ttaagctgca gaagttggtc   840

gtgaggcact gggcaggtaa gtatcaaggt tacaagacag gtttaaggag accaatagaa   900

actgggcttg tcgagacaga gaagactctt gcgtttctga taggcaccta ttggtcttac   960

tgacatccac tttgcctttc tctccacagg tgtccactcc cagttcaatt acagctctta  1020

aggctagagt acttaatacg actcactata ggctagcctc gagaattcac gcgtggtacc  1080

tctagagtcg acccgggcgg ccgcttcgag cagacatgat aagatacatt gatgagtttg  1140

gacaaaccac aactagaatg cagtgaaaaa aatgctttat ttgtgaaatt tgtgatgcta  1200

ttgctttatt tgtaaccatt ataagctgca ataaacaagt taacaacaac aattgcattc  1260

attttatgtt tcaggttcag ggggagatgt gggaggtttt ttaaagcaag taaaacctct  1320

acaaatgtgg taaaatcgat aaggatccgg gctggcgtaa tagcgaagag gcccgcaccg  1380

atcgcccttc ccaacagttg cgcagcctga atggcgaatg gacgcgccct gtagcggcgc  1440

attaagcgcg gcgggtgtgg tggttacgcg cagcgtgacc gctacacttg ccagcgccct  1500

agcgcccgct cctttcgctt tcttcccttc ctttctcgcc acgttcgccg gctttccccg  1560

tcaagctcta aatcgggggc tccctttagg gttccgattt agtgctttac ggcacctcga  1620

ccccaaaaaa cttgattagg gtgatggttc acgtagtggg ccatcgccct gatagacggt  1680

ttttcgccct ttgacgttgg agtccacgtt ctttaatagt ggactcttgt tccaaactgg  1740

aacaacactc aaccctatct cggtctattc ttttgattta taagggattt tgccgatttc  1800

ggcctattgg ttaaaaaatg agctgattta acaaaaattt aacgcgaatt ttaacaaaat  1860

attaacgctt acaatttcct gatgcggtat tttctcctta cgcatctgtg cggtatttca  1920

caccgcatat ggtgcactct cagtacaatc tgctctgatg ccgcatagtt aagccagccc  1980

cgacacccgc caacacccgc tgacgcgccc tgacgggctt gtctgctccc ggcatccgct  2040

tacagacaag ctgtgaccgt ctccgggagc tgcatgtgtc agaggttttc accgtcatca  2100

ccgaaacgcg cgagacgaaa gggcctcgtg atacgcctat ttttataggt taatgtcatg  2160

ataataatgg tttcttagac gtcaggtggc acttttcggg gaaatgtgcg cggaacccct  2220

atttgtttat ttttctaaat acattcaaat atgtatccgc tcatgagaca ataaccctga  2280

taaatgcttc aataatattg aaaaaggaag agtatgagta ttcaacattt ccgtgtcgcc 2340
cttattccct tttttgcggc attttgcctt cctgtttttg ctcacccaga aacgctggtg 2400
aaagtaaaag atgctgaaga tcagttgggt gcacgagtgg gttacatcga actggatctc 2460
aacagcggta agatccttga gagttttcgc cccgaagaac gttttccaat gatgagcact 2520
tttaaagttc tgctatgtgg cgcggtatta tcccgtattg acgccgggca agagcaactc 2580
ggtcgccgca tacactattc tcagaatgac ttggttgagt actcaccagt cacagaaaag 2640
catcttacgg atggcatgac agtaagagaa ttatgcagtg ctgccataac catgagtgat 2700
aacactgcgg ccaacttact tctgacaacg atcggaggac cgaaggagct aaccgctttt 2760
ttgcacaaca tgggggatca tgtaactcgc cttgatcgtt gggaaccgga gctgaatgaa 2820
gccataccaa acgacgagcg tgacaccacg atgcctgtag caatggcaac aacgttgcgc 2880
aaactattaa ctggcgaact acttactcta gcttcccggc aacaattaat agactggatg 2940
gaggcggata aagttgcagg accacttctg cgctcggccc ttccggctgg ctggtttatt 3000
gctgataaat ctggagccgg tgagcgtggg tctcgcggta tcattgcagc actggggcca 3060
gatggtaagc cctcccgtat cgtagttatc tacacgacgg ggagtcaggc aactatggat 3120
gaacgaaata gacagatcgc tgagataggt gcctcactga ttaagcattg gtaactgtca 3180
gaccaagttt actcatatat actttagatt gatttaaaac ttcattttta atttaaaagg 3240
atctaggtga agatcctttt tgataatctc atgaccaaaa tcccttaacg tgagttttcg 3300
ttccactgag cgtcagaccc cgtagaaaag atcaaaggat cttcttgaga tccttttttt 3360
ctgcgcgtaa tctgctgctt gcaaacaaaa aaaccaccgc taccagcggt ggtttgtttg 3420
ccggatcaag agctaccaac tctttttccg aaggtaactg gcttcagcag agcgcagata 3480
ccaaatactg ttcttctagt gtagccgtag ttaggccacc acttcaagaa ctctgtagca 3540
ccgcctacat acctcgctct gctaatcctg ttaccagtgg ctgctgccag tggcgataag 3600
tcgtgtctta ccgggttgga ctcaagacga tagttaccgg ataaggcgca gcggtcgggc 3660
tgaacggggg gttcgtgcac acagcccagc ttggagcgaa cgacctacac cgaactgaga 3720
tacctacagc gtgagctatg agaaagcgcc acgcttcccg aagggagaaa ggcggacagg 3780
tatccggtaa gcggcagggt cggaacagga gagcgcacga gggagcttcc agggggaaac 3840
gcctggtatc tttatagtcc tgtcgggttt cgccacctct gacttgagcg tcgatttttg 3900
tgatgctcgt caggggggcg gagcctatgg aaaaacgcca gcaacgcggc ctttttacgg 3960
ttcctggcct tttgctggcc ttttgctcac atggctcgac agatct 4006